# EUROPEAN PATENT SPECIFICATION

(11) **EP 1 244 345 B1**
(45) Date of publication and mention of the grant of the patent: **02.07.2003**
(21) Application number: 00982810.4
(22) Date of filing: 21.12.2000
(51) Int. Cl.: A01H 1/04

(54) **ENHANCING BIOLOGICAL ACTIVITY OF PLANTS**
ERHÖHUNG DER BIOLOGISCHEN AKTIVITÄT VON PFLANZEN
AMELIORATION DE L'ACTIVITE BIOLOGIQUE DE VEGETAUX

(30) Priority: 29.12.1999 US 475207
(43) Date of publication of application: 02.10.2002
(73) Proprietor: Vitaplant AG, 4108 Witterswil (CH)
(72) Inventor: BUETER, Bernd, CH-4108 Witterswil (CH)
(74) Representative: Ritscher, Thomas, Dr.
(86) International application number: CH0000678
(87) International publication number: WO01049103

(56) References cited:
- WO-A-99/53041
- US-A- 5 770 789
- US-A- 5 817 913
- US-A- 5 869 340

## Description

The present invention generally relates to plants containing physiologically active metabolites, which plants have a proven or presumed positive action on human or animal health and thus are used for pharmaceutical and/or nutritional purposes. More particularly, the present invention relates to a method of enhancing a specific biological activity of a plant containing at least one physiologically active metabolite.

Bioactivity of botanical materials is broadly acknowledged by the scientific and medical communities. For many years, researchers have been able to isolate, identify and synthesize single molecules from plants that produce desirable, specific therapeutic effects in humans or animals. Drugs sold over the counter, such as aspirin deriving from the willow tree, and more recent prescription pharmaceuticals, such as Taxol™ deriving from the yew tree, were developed by identifying the bioactivity in a single molecule.

In recent years there has been an increased interest in therapeutic practices of many cultural groups throughout the world suggesting that multiple molecule cocktails derived from botanical materials and administered in various forms have substantial bioactivity that may be superior or at least comparable in effectiveness compared to existing pharmaceuticals. Although profound evidence is available for positive health effects of certain plants containing those physiologically active metabolites, the way of action, i.e. the way by which the totality of the biologically active compounds lead to the observed positive health effects, are usually unknown. Such lack of knowledge with regard to the way of actions and active principles, respectively, is primarily caused by the given complexity due to the plurality of components, i.e. primary and secondary metabolites, contained in those plants and its large variation in composition. It is known that the composition of physiologically active metabolites and their concentrations in those plants can vary significantly depending either on environmental factors, such as location of plant growth and harvesting conditions, and, in particular, on genetic factors.

The aforementioned variations in composition of the biologically active compounds generally lead to variations in the desirable biological activity of those plants causing problems diminishing, in particular, the economic incentive for further exploitation of those plants. For reason of simplicity, the term "physiologically active plants" and its further abbreviation "PA-plants" is used herein to refer to plants containing physiologically active metabolites.

The increasing quality standards applied to phytopharmaceuticals, nutraceuticals and health foods require an assurance for quality, efficacy and stability of the final products derived from PA-plants. Since the biological activity of PA-plants varies significantly depending upon the genetic and environmental parameters, as indicated above, these high quality standards need an efficient standardization procedure. For obvious reasons, efficient standardization procedures require the knowledge of the active principles. Due to a general lack of knowledge with regard to the active principles, as already mentioned, standardizing on the totality of the biological active compounds is typically replaced by standardizing on so called "marker substances" or "markers", i.e. specific known constituents of the plant, whose relevance for the therapeutical use has, however, not been generally proven. Standardizing on marker substances would and is only suitable and reliable if the content of these marker substances was and is correlated to the content of the actual active compounds and though to the actual given biological activity.

As indicated, the presently dominating way of standardization of phytopharmaceuticals via markers is - due to the mentioned lack of correlation between markers and the biological activity of the PA-plant - not suitable as a mean for quality assurance. Therefore, such a standardization via markers can not assure that the final product derived from the PA-plant has a stable quality and a stable physiological effect even though the content of the marker substance in the standardization procedure is stable.

A further problem of prior art standardization methods is the late stage at which such a standardization takes place. Taking into consideration that it starts generally after the raw plant material has been gathered and generally has been processed, it appears that only a very limited degree of standardization can be accomplished. Since the raw plant material frequently derives from plants, which are gathered from different and/or usually unknown sources and whose genetic constitution is unknown and undefined, and which raw plant material has generally been processed, i.e. harvested, cleaned, cut, dried and stored, under different and usually unknown conditions, such raw plant material displays significant variations in composition and concentration of the physiologically active metabolites. An eventual mixing and/or combining of those raw plant materials and/or fractions therefrom inevitably will lead to an uncontrolled variation in composition and concentration of the physiologically active metabolites between such mixtures or combinations, and thus to an uncontrolled variation in the desired biological activity and activities respectively.

Therefore, there is a need for innovative strategies which allow the development of plants with an optimized profile of biologically active ingredients.

Undesirable variations caused, in particular, by genetic and environmental factors can only be avoided if the standardization procedures are applied at an earlier stage, i.e. before and during the generation of the raw plant material, i.e. the material which is incorporated in phytopharmaceuticals, nutraceuticals and health foods. Thus, for a real and reliable standardization, the plant which eventually gives the plant raw material has to be standardized, i.e. the generic constitution of the plant has to be defined, and the conditions under which the plant is cultivated, grown and processed have to be standardized.

Accordingly, it is an object of the present invention to provide for a method of enhancing a specific biological activity of a plant, which method allows to select, breed and/or otherwise develop plants with improved and optimized contents of physiologically active substances, i.e. with an improved and optimized composition profile of physiologically active metabolites and with an improved and optimized concentration profile of these physiologically active compounds, and thus with an enhanced and optimized specific biological activity and activities respectively, notably a method of enhancing a specific biological activity of a plant, which method allows to determine, to select and/or develop an optimized processing procedure for the production of plants having an increased biological activity for a specific indication.

US5770789, US5869340, and US5817913 each disclose a method of enhancing a specific biological activity of a plant containing at least one physiologically active metabolite, comprising the steps of (a) collecting a plurality of samples of said plant; (b) cultivating each of said samples under at least a first set of defined conditions to produce mated plants of each of said samples; (c) processing each of said matured plants under at least a first set of defined conditions to obtain plant material of each of said matured plants; (d) determining said specific biological activity of each of said plant material by means of at least one biological assay to obtain a racking of said plant materials indicating plant materials with a higher specific biological activity; and (e) selecting said plant materials with a higher specific biological activity to identify elite plants.

Thus, the present invention provides for a method as defined in claim 1 and its dependent claims 2-7 and provides a tool for the development/breeding and/or processing/production of PA-plants with improved contents of those physiologically active substances; the development/breeding and/or processing/production of such improved PA-plants is based on the use of biological assays; either *in vitro* or *in vivo*, however, *in vitro* being preferred.

Moreover, the inventive method described herein permits to reduce and/or to exclude variations in composition of the active metabolites and the actual biologically active substances respectively since standardization is carried out at the earliest possible step, i.e. the definition of the plant, its genetic constitution, and the conditions under which the plant is grown, cultivated, multiplied and processed. PA-plants developed with the inventive method and methods respectively consistently will result in final products with a high degree of biological activity without any further measures required on the extraction level, such as fractionation and re-mixing of fractions or others. Thus, the inventive method represents an efficient way towards truly standardized plants for use in highly active plant based products, such as extracts, powders, fresh plants, dried or otherwise preserved plants or parts of them, as well as highly active herbal medicinal products.

As indicated, the plants used for the present invention have at least one proven or presumed impact on human or animal health, typically at least one positive action on human or animal health. Such an impact on human or animal health of a plant is termed herein "specific biological activity" of the plant. Thus, the choice of the at least one bioassay used in the present invention to determine the specific biological activity of the plant and plant material respectively is, though, guided by that proven or presumed impact on human or animal health of the plant.

In the context of the present invention the term "samples" stands for individual plants, typically belonging to the same plant species and being either detived from field excursions, being commercially available or being obtained by other sources. It is obvious, however, that specific parts or organs of individual plants, which are suitable for propagation, can also be used for the present invention in order to provide samples in accordance with the present invention. In this case, then, a propagation and multiplication of those parts or organs is typically effected prior to the cultivation of the samples.

The cultivation of each of the samples is typically effected under a first set of defined conditions. In the context of the present invention, a "set of defined cultivation conditions" indicates that the cultivation for each of the samples, variants or elite plants is effected under the same defined conditions. The well-defined cultivation with respect to the conditions of each of the samples, variants or elite plants in accordance with the present invention is particularly advantageous since it minimizes or even excludes impacts of environmental factors on the composition and concentrations of the physiologically active metabolites contained then in the matured plants and, thus, allows the comparison between the different samples, variants or elite plants, which comparison is essentially based on their genetic constitution. The minimization and/or exclusion of variation caused by environmental factors is crucial and a first prerequisite for a reliable identification, selection, breeding, and thus development of plants and matured plants respectively having an enhanced specific biological activity. It is obvious, however, that the at least first set of defined conditions can be different for the cultivation of samples, variants and for the cultivation of elite plants. Moreover, the sets of defined cultivation conditions can also vary in depending of the particular plant used. The artisan is well aware of the plurality of parameters defining such a set of cultivation conditions; by way of example, the soil type, planting density, fertilization, irrigation, light/sun exposure (shading), method of weed control, time to harvest, developmental stage of plant at harvest and harvested parts of plants are mentioned herein.

The term "matured plants" as used herein, refers to plants, which are generated in a cultivation step in accordance with the present invention. Thus, matured plants are plants having an increased biomass with respect to the samples used at the beginning of the cultivation. It is to be noted, however, that a "matured plant" as used herein refers to any developmental stage of a plant generated in a inventive cultivation step having an increased biomass, at which developmental stage the plant is capable of being further processed in accordance with the invention.

The processing of the matured plants in accordance with the invention is typically effected under at least one, i.e. a first set of defined conditions. In analogy to the above-mentioned set of defined cultivation conditions, a "set of defined processing conditions" indicates that the processing for each of the matured plants is effected under the same defined, and, thus, comparable conditions. Typical parameters of such a set of processing conditions are light exposure after harvest, type of washing device, washing time, water pressure during washing, type of cutting device, cutting size, type of dryer, drying temperature, drying time or period, way of packing, storage conditions, and the way and applied conditions for extraction of the plant or parts of it, such as the solvent used for extraction (e.g. water, alcohol, alcohol-mixtures, supercritical carbon dioxide).

Accordingly, the processing of each of the matured plants under the same defined conditions minimizes or even excludes impacts caused the totality of processing parameters on the composition and concentrations of the physiologically active metabolites contained then in the plant material. Such a minimization and/or exclusion is, as it is in the case of the cultivation steps according to the invention, crucial and another prerequisite for a reliable identification, selection, breeding, and thus development of plants and elite plants respectively having an enhanced specific biological activity.

In the context of the present invention the term "plant material" refers to any type and form of a plant or a selected part of a plant deriving from a matured plant, as defined above, which type and form is suitable to be employed directly or after being processed in bioassays in accordance with the present invention. Thus, examples of different types and forms of plant material in accordance with the present invention includes but is not limited to the fresh plant or selected parts of it, an aqueous or organic extract of the whole plant or selected parts of it, a powder of the dried or freeze-dried whole plant or a selected part of it, a frozen product prepared from the fresh plant or parts of it, and preferably a juice, drink, sap obtained by squeezing, vortexing or otherwise treating the whole plant or a selected part of it.

The term "elite plants" as used herein refers to those plants and matured plants respectively, the plant materials of which are identified and selected as the ones having a higher specific biological activity as determined by means of biological assays. Even if a wide array of relevant biological assays, e.g. assays based on enzymatic activity, cellular based assays, receptor binding assays or even animal based assays, is known, the person skilled in the art is capable of choosing the appropriate biological assay to determine the specific biological activity of the plant and plant material in accordance with the invention.

Typically, the plant is selected from Hypericum perforatum (St. John's Wort), Valeriana officinalis, Piper methysticum (Kava Kava), Vitex agnus castus (Chaste tree), Petasites hybridus (Butterbur), Cynara scolymus (Artichoke), Cimicifuga racemosa (Black Cohush), Passiflora incarnata (Passionflower), Boswellia ssp. (Incense) or Isatis tinctoria (Woad). However; any other plant having a proven or presumed positive action on human or animal health and thus being used or being a potential candidate for pharmaceutical and/or nutritional purposes can be used for the present invention.

The present invention allows to create, i.e. modify, particularly to mutate, to intermate, to fuse, to genetically transform existing plants in a way that they will display an improved biological activity, in particular, within the same plant species. However, it is well within the scope of the present invention to use plants from different plant species or transfer genes of microbial (including fungi and viruses), plant or animal origin to a target plant in order to create and develop new plants with enhanced specific biological activity. By way of example, it is well within the scope of the present invention to combine plants of *Hypericum perforatum* and *Hypericum maculatum* and assay the offspring and plant material respectively for its biological activity.

The multiplication of each of the elite plants can be effected by any possible propagation procedures known to the person skilled in the art. The preferred multiplication and propagation procedure is, however, a true-to-type multiplication, e.g. by a vegetative propagation.

With respect to the production of variants of the elite plants in accordance with the present invention, such production of variants is not limited to a particular production procedure and any method available such as intermating, mutation induction, haploidization, polyploidization, protoplast fusion, genetic transformation or other can be applied. Typically, a plurality of variants of each of the elite plants is produced. The exact number of variants produced and the exact number of elite plants from which variants are produced is appropriately chosen for the particular case, i.e. the type of plants used in the present invention, and is, thus, within the knowledge of the artisan.

According to a preferred embodiment of the first general embodiment of the present invention, the method of enhancing a specific biological activity of a plant further comprises the step of (ii) repeating at least once a sequence of said steps (g), (h) and (i) of claim 1 with the elite plants of each previous sequence. The step (ii) is typically effected after having effected said step (i) of claim 1 and prior to the performance of said step (k) of claim 1. This preferred embodiment of the present invention is particularly advantageous since it might allow to further enhance and improve the specific biological activity of the plant. Thus, a second generation of variants of the second generation of elite plants are produced, then cultivated under at least a first set of defined conditions to produce matured plants thereof, followed by processing each of the so obtained matured plants leading to the corresponding plant material. Then, the specific biological activity of each of the plant material is determined by at least one appropriate biological assay to obtain a ranking of the investigated plant materials, and finally the plant materials and the next generation of elite plants, respectively, having a higher specific biological activity are selected and identified, as well as multiplied. The cultivation steps of the preferred embodiment again ensures the reduction or even exclusion of impacts of environmental on the composition and concentrations of the physiologically active metabolites contained then in the matured plants and , thus, again allows the comparison between this generation of different samples, variants or elite plants, which comparison is essentially based on their genetic constitution. Such a comparison and selection mainly based on genetic factors is furthermore guaranteed by the same defined set of processing conditions. The advantages connected with the use of biological assays to determine the specific biological activity have already been mentioned herein and its essentiality and importance for the present invention is herewith again emphasized.

Preferably, the genetic fingerprint of each of said elite plants is determined, which is preferably effected before and after multiplication of said elite plants. This allows to control the quality of the multiplication steps.

In a further preferred embodiment of the invention, the elite plants are cultivated by at least a first and a second set of defined conditions, preferably by a plurality of sets of defined conditions. Typically, the first general method of the present invention further comprises the step of (1) repeating at least once a sequence of said steps (c)-(f) and (k) of claim 1 with said matured plants of said elite plants of each previous sequence. Therefore, the present invention provides for a method which allows to select the most appropriate plant cultivation and processing technologies of the selected elite plants, i.e. to select technologies which lead to optimum contents of biologically active compounds in the elite plants by using biological assays. Such plants can then be manufactured into high quality, efficient and stable final products which fulfill the legal requirements of the registration authorities and the expectations of the consumers. The plants obtained according to the inventive method are tailor-made with regard to their final intended use in a phytoparmaceutical, nutraceutical, pharmaceutical or health food product. Such tailor-made, genetically defined plants reveal an improved quality in comparison with non defined plants or plants defined by their content of a marker substance.

In a third general embodiment, the present invention provides for a method of selecting elite plants comprising the steps of: (a) collecting a plurality of samples of a plant; (b) cultivating each of said samples under at least a first set of defined conditions to produce matured plants of each of said samples; (c) processing each of said matured plants under at least a first set of defined conditions to obtain plant material of each of said matured plants; (d) determining said specific biological activity of each of said plant material by means of at least one biological assay to obtain a ranking of said plant materials indicating plant materials with a higher specific biological activity; (e) selecting said plant materials with a higher specific biological activity to identify elite plants;

In a fourth general embodiment, the present invention provides for a method of selecting elite plants comprising method of selecting elite plants comprising the steps of (a) collecting a plurality of samples of a plant; (b) cultivating each of said samples under at least a first set of defined conditions to produce matured plants of each of said samples; (c) processing each of said matured plants under at least a first set of defined conditions to obtain plant material of each of said matured plants; (d) determining said specific biological activity of each of said plant material by means of at least one biological assay to obtain a ranking of said plant materials indicating plant materials with a higher specific biological activity; (e) selecting said plant materials with a higher specific biological activity to identify elite plants; wherein, for the purpose of enhancing the specific biological activity of said elite plants said method comprises the steps of (f) producing variants of said elite plants; (g) cultivating each of said variants under at least a first set of defined conditions to produce matured plants of each of said variants; (h) repeating said steps (c)-(f) with said matured plants of each of said variants; (i) cultivating said elite plants under at least a first set of defined conditions to produce matured plants of said elite plants.

In a fifth general embodiment, the present invention provides for a method of producing elite plants comprising the steps of (a) collecting a plurality of samples of said plant; (b) cultivating each of said samples under at least a first set of defined conditions to produce matured plants of each of said samples; (c) processing each of said matured plants under at least a first set of defined conditions to obtain plant material of each of said matured plants; (d) determining said specific biological activity of each of said plant material by means of at least one biological assay to obtain a ranking of said plant materials indicating plant materials with a higher specific biological activity; (e) selecting said plant materials with a higher specific biological activity to identify elite plants; (f) multiplying at least one of said elite plants; (g) producing variants of said elite plants; (h) cultivating each of said variants under at least a first set of defined conditions to produce matured plants of each of said variants; (i) repeating said steps (c)-(f) with said matured plants of each of said variants; and (k) cultivating said elite plants under at least a first set of defined conditions to produce matured plants of said elite plants.

The present invention will be explained, without limitation, in more detail by reference to the following examples and the enclosed drawings illustrating some preferred embodiments, in which
Fig. 1 shows the results of radioligand binding studies, i.e. specific binding values of ³H-naloxone to human K-opioid receptor, given in %, of selected *Hypericum perforatum* accessions while varying the concentrations of the extracts;
Fig. 2 shows the results of radioligand binding studies, i.e. specific binding values of ¹²⁵I-sauvagine to human CHR₁ receptor, given in %, of selected *Hypericum perforatum* accessions while varying the concentrations of the extracts;
Fig. 3a shows relative amounts of different pharmalogically relevant compounds in the *Hypericum perforatum* genotype VITAN;
Fig. 3b shows relative amounts of different pharmalogically relevant compounds in the *Hypericum perforatum* genotype HP57-II.

### Example 1:

As indicated above, phytopharmaceuticals are generally standardized on markers, whose relevance for the therapeutical use has usually not been proven. In Example 1, the use of biological assays *in vitro* such as radioligand binding studies to receptors is applied, by way of example, in the inventive method to compare the biological activities of different *Hypericum perforatum* accessions. In accordance with the present invention, in Example 1 the use of biological assays *in vitro* for the selection and identification of a *Hypericum perfotatum* genotype with improved antidepressant effects is described. By way of example, the use of radioligand binding studies with receptors expressed with the Semliki Forest virus system is described. The potency of different *Hypericum perforatum* accessions to inhibit the binding to selected receptors of the central nervous system is compared.

As it is case for many other virus systems, the use of the Semliki Forest virus system also allows to study the affinity of plant extracts to receptors as well as to enzymes important in other indications such as hormonal disorders, gastrointestinal diseases, inflammation and others. Briefly, the Semliki Forest virus expression system is composed of a pSFV vector carrying the recombinant receptor and a helper plasmid.

For generation of virus particles, *in vitro* RNA is transcribed with SP6 RNA polymerase and electroporated into baby hamster kidney (BHK) cells (cf. Berglund, P., Sjöberg, M., Garoff, H., Atkins, J.G., Sheahan, B.J. and Liljeström, P., Bio/Technology 11 (1993), 916-920; Lundstrom, K., Mills, A., Buell, G., Allet, E., Adami, N. and LiIjestrom, P., Eur. I Biochem. 224 (1994), 917-921). After 24 h, the recombinant virus particles are collected. Chinese hamster ovary cells (CHO) of 80-90% confluency are then infected with the SFV virus at a multiplicity of infection of 10. The infected CHO cells 16-48 hr post-infection are lysed and the fractions containing the receptors or enzymes are isolated. In the case of radioligand binding studies, the potential of plant extracts to compete for the binding of a radio-labelled ligand will be performed.

The aforementioned biological activity tests in accordance with the invention allow to efficiently screen a large number of genetically diverse accessions in order to select the optimal genotypes according to the pharmaceutical activity. As already indicated, the inventive method, then, can develop and create respectively new accessions by breeding measures, such as intermating, mutation induction, haploidization, polyploidization, genetic transformations, protoplast fusion, crossing or others. Those newly bred accessions can then be screened alike according to the invention to select and identify again the elite accessions from the newly bred accessions, which elite accessions have a higher specific biological activity and activities respectively.

Extracts of the traditional medicinal herb *Hypericum perforatum*, L., St. Johns wort, primarily known for its antidepressant activity, are usually standardized on the naphthodianthrone hypericin. Although hypericin has been shown to affect several receptors of the central nervous system such as sigma, serotonin (5-HT), opioid and corticotropin releasing hormone (CRH) receptors (cf. Raffa, R.B., Life Sci. 62 (1998), 265-270; Simmen, U., Berger, K., Burkard, W., Schaffher, W., Lundstrom, K., J. Recept. Signal Transduct. Res. 19(1-4) (1999), 59-74), it seems likely that hypericin is not the only active principle. Other components such as the acylphloroglucinol hyperforin, the proanthocyanidins and the flavonoids have further been described to be involved in the antidepressant effect (cf. Butterweck, V., PhD Thesis, Münster (1997); Chattejee, S.S., Bhattacharya, S.K., Wonnemann, M., Singer, A. and Müller, W.E., Life Sci 63 (1998), 499-510; Simmers, U., Berger, K., Burkard, W., Schaffher, W., Lundstrom, K., J. Recept. Signal Transduct. Res. 19(1-4) (1999), 59-74). Hyperforin was shown to potently inhibit neuronal reuptake of monoamines and amino acids, while the proanthocyanidins may increase the solubility of the hypericins. A weak affinity to the estrogen receptor has further been shown for the biflavonoid biapigenin. In addition to hyperforin, other hyperforin-like constituents may also play a certain role (cf. Bhattacharya, S.K., Chakrabarti, A. and Chatterjee, S.S., Pharmacopsychiat. 31 (Suppl.) (1998), 22-29; Dimpfel, W., Schober, F. and Mannel, M., Pharmacopsychiat. 31 (Suppl.) (1998), 30-35; Simmen, U., Berger, K., Burkard, W., Schaffher, W., Lundstrom, K., J. Recept. Signal Transduct. Res. 19(1-4) (1999), 59-74).

Several mechanisms of action mainly affecting the metabolism of central monoamines have been proposed to explain the antidepressant effect of *Hypericum perforatum*. According to studies *in vivo,* adaptive changes such as the downregulation of β-adrenoceptors as well as changes of serotonergic as well as dopaminergic systems have been observed (cf. MüIler, W.E. and Schäfer, C., Deutsche Apotheker Zeitung. 136 (1996), 17-24; Winterhoff, H., Butterweck, V., Nahrstedt, A., Gumbinger, H.G., Schulz, V., Erping, S., Bosshammer, F. and Wieligmann, A. in: D. Loew and N. Riehbrock (Eds.), Phytopharmaka in Forschung und klinischer Anwendung. Steinkopf Verlag, Darmstadt, (1995), pp. 39-56; Bhattacharya, S.K., Chakrabarti, A. and Chatterjee, S.S., Pharmacopsychiat. 31 (Suppl.) (1998), 22-29). In addition, *in* vitro-studies have shown the effects of extracts and constituents of *Hypericum perforatum* on numerous CNS receptors as well as the inhibition of reuptake of both monoamines and amino acids (cf. Baureithel, K.H., Berger Bueter, K., Engesser, A., Burkard, W. and Schafffier, W. Pharmac. Acta Helvetiae 72 (1997), 153-157; Wonnemann, M., Schäfer, C. and Müller, W.E., Pharmacopsychiat. 30 (1997), 237; Chatterjee, S.S., Bhattacharya, S.K., Wonnemann, M., Singer, A. and MüIler, W.E., Life Sci 63 (1998), 499-5 10; Raffa, R.B., Life Sci. 62 (1998), 265-270; Simmen, U., Berger, K., Burkard, W., Schaffher, W., Lundstrom, K., J. Recept. Signal Transduct. Res. 19(1-4) (1999), 59-74). The majority of these receptors such as the α- and β-adrenoceptors, 5-HT, sigma, opioid, CRH and dopamine receptors are G-protein coupled receptors, which may effect adenylate cyclase activity. In addition, ionotropic receptors such as the GABA_{A} and NMDA channel may regulate the flow of chloride through the plasma membrane, while cytosolic steroid receptors like the estrogen receptor may directly regulate transcription levels.

Here, radioligand binding experiments *in vitro* on κ-opioid and the CRH₁ receptors were performed in order to study the affinities of *Hypericum perforatum* extracts to these receptors. Binding to both receptors has previously been shown to be affected by extracts and constituents of *Hypericum perforatum* (Simmen, U., Schweitzer, C, Burkard, W., Schaffner, W. and Lundstrom, K., Pharmac. ActaHelv. 73 (1998), 53-56; Simmen, U., Berger, K., Burkard, W., Schaffher, W., Lundstrom, K., J. Recept. Signal Transduct. Res. 19(1-4) (1999), 59-74; Simmen, unpublished results). Opioid receptors are known to be involved in changes of mood behaviour in addition to their analgesic action (cf. Martin, W.R., Pharmacol. Rev. 35 (1983), 283-323; Pasternak, G.W., Clin. Neuropharmacology 16 (1993), 1-18). CRH receptors have been proposed to play a certain role in antidepressant mechanisms, since depressions are usually accompanied by an oversecretion of hypothalamic CRH as well as elevated cortisol levels (Sternberg E.M. and Gold P.W., Special Issue, Vol. 7, No. 1. (1997), pp.8-15). The κ-subtype of the opioid receptors was transiently expressed in Chinese hamster ovary (CHO) cells using the Semliki Forest virus (SFV) expression system (cf. Simmen, U., Schweitzer, C, Burkard, W., Schaffner, W. and Lundstrom, K., Pharmac. ActaHelv. 73 (1998), 53-56). The use of the SFV system allows the production of large quantities of receptors useful for both binding and functional studies *in vitro* (cf. Lundstrom, K., Focus 18 (1997), 53-56). Consequently, SFV expressed receptors generally provide improved binding characteristics in comparison to receptor material isolated from brain tissues. The CRH₁ receptor was stably expressed in human embryonic kidney (HEK) cells (cf. Gottowik, J., Goetschy, V., Henriot, S., Kitas, E., Fluhman, B., Clerc, R. G., Moreau, J.-L., Monsma, F.J. and Kilpatrick, G.J., Neuropharmacology 3 6 (1997), 1439-1446).

**Plant Materials and Extraction.** The effect of four *Hypericum perforatum* accessions (VITAN, TOPAZ-FEL, HP57-II, HP49) that differ in their secondary metabolite profile were compared on the binding of ³H-naloxone and ¹²⁵I-sauvagine to opioid and CRH receptors, respectively. Genetic factors have previously been shown to strongly affect the content of secondary metabolites in *Hypericum perforatum* cultivars (cf. Büter, B., Orlacchio, C., Soldati, A. and Berger, K., Planta Medica 64 (1998), 431-437).

All plants used in the reported experiments were grown at the same site (Witterswil, Switzerland) under the same conditions (planting date, planting density, fertilization, etc.). Dried stems, leaves and flowers, i.e. the upper part [30 cm] of the plants, from cultivated *Hypericum perforatum* were collected in the stage of full flowering, dried for 48 hrs at 40°C, and extracted with methanol and subsequently with aceton (150 g dry weight/l solvent) for 48 hrs at 40'C.

The extracts were dried under reduced pressure and resuspended with methanol to a final concentration of 10 mg extract/ml. Extracts were further diluted with methanol in order to maintain a constant methanol concentration in the binding assays. The methanol concentration in the assay was controlled to ensure that the binding was affected less than 5%.

**HPLC-Analysis.** HPLC analysis of *Hypericum* extracts was carried out on an analytical Lichrospher RP-select B column (5 µm, 4 x 250 mm) using a HPLC-Waters equipment coupled to photodiode array detection (Waters 911). The column was equilibrated with 19% acetonitril, 80% water and 1% phosphoric acid (solution A) and eluted with a linear gradient of 59% acetonitril, 40% methanol and 1% phosphoric acid (solution B) according to the following administration: 0-22 min, 0-50% B, 22-37 min, 50-100% B, 37-67 min, 100% B. Constituents were identified by comparing retention times of standard compounds as well as UV spectra analysis.

**Receptors.** The κ-opioid receptor cDNA was subcloned as a *Bam* HI fragment from the pcDNA3 vector (Invitrogen) into the pSFV1 vector and the right orientation of the insert confirmed by restriction analysis to obtain the pSFV1-κ-Opioid-R construct. For generation of the receptor virus stock, *in vitro* RNA was transcribed with SP6 RNA polymerase from the plasmid carrying the recombinant receptor, pSFV3-LacZ, and pSFV-Helper2 and electroporated into Baby Hamster kidney (BHK) cells (cf. Berglund, P., Sjöberg, M., Garoff, H., Atkins, J.G., Sheahan, B.J. and Liljeström, P., Bio/Technology 11 (1993), 916-920; Lundstrom, K., Mills, A., Buell, G., Allet, E., Adami, N. and LiIjestrom, P., Eur. I Biochem. 224 (1994), 917-921). After 24 h, the recombinant virus particles were collected and the titre of the SFV-LacZ virus stock was determined as described previously (cf. Lundstrom, K., Mills, A., Buell, G., Allet, E., Adami, N. and Liljestrom, P., Eur. I Biochem. 224 (1994), 917-921). Because the titre for the pSFV1-κ-Opioid-R virus stock could not be obtained directly, comparison to the SFV-LacZ virus stocks had to be made. As all the virus stocks were packaged in parallel, we assume the titres to be of the same magnitude. This assumption was confirmed by metabolic labelling with [³⁵S]-methionine (NEN) 5 h post-electroporation followed by autoradiography.

CHO cells of 80-90% confluency were infected with the SFV- κ -Opioid-R and SFV-LacZ virus stocks at a multiplicity of infection of 10 and pulse-labelling with [³⁵S]-methionine carried out 16 h post-infection. Protein expression was verified by SDS-PAGE followed by autoradiography.

The human CRH₁ receptor was generated by PCR using a human cerebellum cDNA library as template as described previously (cf. Gottowik, J., Goetschy, V., Henriot, S., Kitas, E., Fluhman, B., Clerc, R. G., Moreau, J.-L., Monsma, F.J. and Kilpatrick, G.J., Neuropharmacology 3 6 (1997), 1439-1446). The cDNA insert was then amplified by PCR and subcloned into the pAMP1 vector and subsequently into the pCEP4. The pCEP4-CRH₁ construct was transfected into HEK-293 cells adapted for suspension culture.

CHO infected T 175 flasks with recombinant SFV-κ-Opioid-R particles 16-48 hr post-infection were briefly washed with 5 mM Hepes buffer pH 7.4, 2 mM EDTA and lysed in the same buffer for 20 min at 4°C. The lysed cells were transferred to 10 ml centrifuge tubes, spun at 40'000 g for 15 min and resuspended in 50 mM Tris/HCl buffer pH 7.8, 1 mM EGTA and 5 mM MgCl₂ using a Polytron homogeniser. After centrifugation at 40'000 g for 15 min, the pellet was stored at -80°C. Immediately before starting the binding assay, membranes were resuspended in Tris/HCl buffer as described above. Protein concentration was determined by the BCA method (cf. Smith, P.K., Krohn, R.I., Hermanson, G.T., Mallia, A.K., Gartner, F.H., Provenzano, M.D., Fujimoto, EX., Gocke, N.M., Olson, B.J. and Klenk, D.C., Anal. Biochem. 150 (1985), 76-85) with bovine serum albumin as the standard.

HEK cells were pelleted after two washing steps and resuspended in ice cold 50 mM Tris buffer pH 7.0, 2 mM EGTA, 10 mM MgCl₂. This homogenate was centrifuged at 100'000 g for 60 min. The resulting pellet was resuspended in the same buffer to obtain a concentration corresponding to 10⁷ cells/ml and the aliquots stored at -80°C.

**Radioligand Binding Studies.** Radioligand binding experiments are considered as fundamental to study the molecular basis of the interaction of a drug to its target site. In such experiments the displacement of a radio-labelled ligand from its binding site is determined in the presence of a given drug. The potency of displacement expressed as IC₅₀ value is determined as 50% inhibition of the specific binding. Specific binding represents the difference between total and non-specific binding. In the case of membrane bound receptors, ligand-receptor complexes may be separated from free ligand molecules by rapid filtration. The total binding represents the total radioactivity bound to the filter. Non-specific binding represents the radioactivity bound to the filter in the presence of a large excess of a selected agonist/antagonist. The total binding should not exceed more than 10% of the total added amount of radioligand. Determination of incubation conditions such as the concentration of the receptor and the ligand as well as incubation time and temperatur is necessary to achieve reliable data.

Binding to κ-opioid receptor was conducted in triplicates in a total volume of 500 µl 50 mM Tris/HCl buffer pH 7.8, 1 mM EGTA and 5 mM MgCl₂ with [N-allyl-2,3-³H] naloxone (54.6 Ci/mmol; NEN) as ligand at a final concentration of 3.6 nM and 4 µg protein. To determine non-specific binding, naloxone at a final concentration of 10 µM was used. Binding was terminated after 1 hr at RT by rapid filtration with GF/C filters under reduced pressure and three washes with cold Tris/HCl pH 7.4 buffer. Radioactivity on filters was determined by liquid scintillation counting (1214 Rackbeta, LKB, WALLAC).

Competition binding assays with ¹²⁵I-labeled sauvagine (20 Ci/mmol, Amersham) on membranes of pCEP4-CRH₁ transfected HEK-293 cells incubated in wheatgerm agglutinin SPA beads (Amersham, 5 mg SPA/ml membrane suspension) were performed in triplicates in a total volume of 300 µl in 50 mM Tris buffer (pH 7.4) containing 2 mM EGTA, 10 mM MgCl₂ and 2.3 mg/l aprotinin. Non-specific binding was determined in the presence of 300 nM human urocortin. After incubation for 1 hr at RT bound radioactivity was separated by centrifugation for 5 min at 3000 rpm and counted for 5 min. Specific binding of different concentrations of extracts and compounds was plotted and curve fittings were performed.

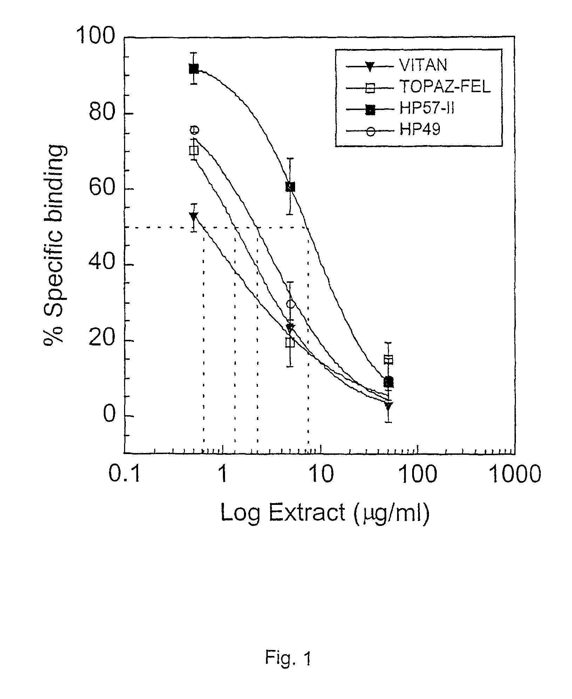

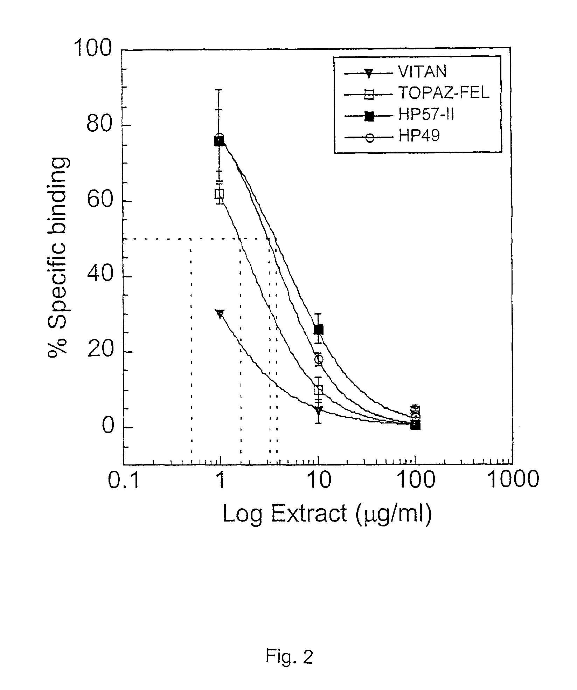

In Example 1 extracts of four accessions of *Hypericum perforatum* (VITAN, TOPAZ-FEL, HP57-II, HP49) were studied concerning their effects on the binding of naloxone and sauvagine to κ-opioid and CRH₁ receptors, respectively. Competition binding studies were performed to determine the potency of displacement of the radioligands (cf. Fig. 1 and Fig. 2). Extract concentrations of 50% binding inhibition (IC₅₀ values) were graphically determined and are shown in Table 1.

**Table 1:**

| Effects of genotypes *of Hypericum perforatum* on the binding of ³H-naloxone and ¹²⁵I-sauvagine to κ-opioid and CRH₁ receptors, respectively | | |
|---|---|---|
| | *IC*_{*50*}*-values (µg*/*ml)* | |
| | κ-Opioid-R | CRH₁-R |
| VITAN | 0,5 | 0,6 |
| TOPAZ-FEL | 1,5 | 1,3 |
| HP57-II | 4 | 8 |
| HP49 | 3 | 2 |

The most potent affinity to both κ-opioid and CRH₁ receptors was observed for the accession VITAN. This extract inhibited the binding of naloxone and sauvagine to κ-opioid and CRH₁ receptors approximately 8 to 12 times more potently than the extract of HP57-II, respectively.

HPLC analysis was performed to quantify the amounts of constituents present in the extracts of different *Hypericum perforatum* accessions (cf. Table 2).

**Table 2:**

| Secondary metabolite contents of 4 different *Hypericum perforatum* accessions | | | | |
|---|---|---|---|---|
| mg/100mg (dried flowers) | Vitan | Topaz | HP 49 | HP 57 |
| Total Hypericins | 0.49 | 0.43 | 0.42 | 0.34 |
| | | | | |
| Hypericin | 0.24 | 0.07 | 0.15 | 0.07 |
| | | | | |
| Pseudohypericin | 0.25 | 0.36 | 0.27 | 0.26 |
| | | | | |
| Hyperforin | 1.13 | 0.07 | 2.52 | 0.43 |
| | | | | |
| Rutin | 0.6 | 0.6 | 0.85 | 0.97 |
| | | | | |
| Hyperosid | 2.17 | 1.36 | 1.19 | 1.08 |
| | | | | |
| Isoquercitrin | 0.5 | 0.21 | 0.45 | 0.32 |
| | | | | |
| Quercitrin | 0.41 | 0.2 | 0.47 | 0.2 |
| | | | | |
| Quercetin | 0.09 | 0.08 | 0.03 | 0.05 |
| | | | | |
| Biapigenin | 0.79 | 0.73 | 0.9 | 0.79 |
| | | | | |
| Amentoflavon | 0.02 | 0.02 | 0.02 | 0.02 |

The main components such as the flavonoid glycosides, the biflavonoids and the hypericins were identified and quantified comparing corresponding standards and their retention times. The extracts contained only small amounts of hyperforin and other lipophilic compounds (data not shown). Since these compounds are known to be relatively unstable under light and oxygen exposure, they may be degraded during the extraction procedure.

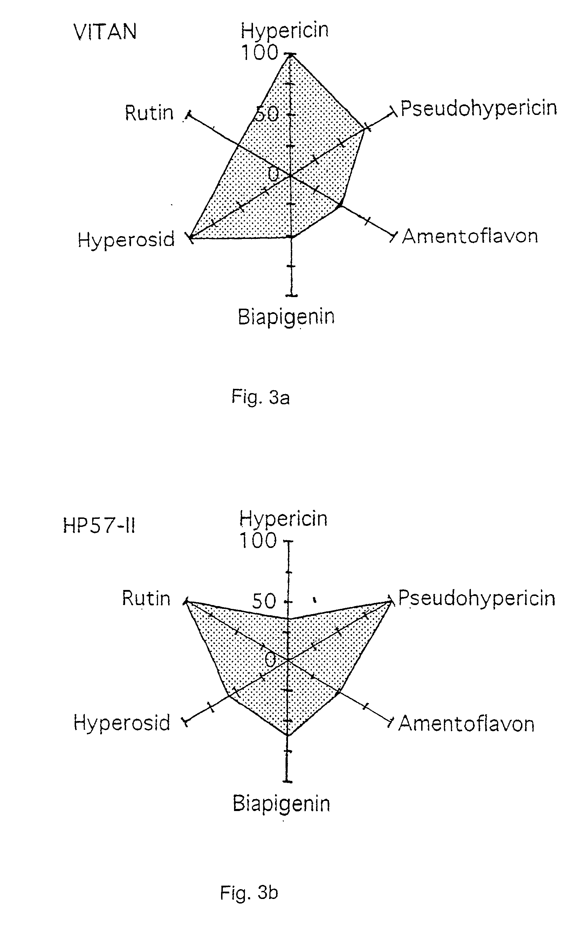

The relative amounts of the main ingredients of the genotypes VITAN and HP57-II are shown in Fig. 3a and Fig. 3b. Both extracts contained similar amounts of the biflavonoids amentoflavon and biapigenin, while the content of the flavonoid glycosides such as rutin and hyperosid differed by a maximum of a factor two. The genotype HP57-II that exhibited the least potent effects *in vitro,* contained approximately three times less hypericin, but little more pseudohypericin than VITAN. The hypericins have previously been shown to affect both opioid and CRH receptors, whereas the flavonoids were ineffective (cf. Simmen, U., Berger, K., Burkard, W., Schaffher, W., Lundstrom, K., J. Recept. Signal Transduct. Res. 19(1-4) (1999), 59-74). Hence, the distinct amounts of ingredients can not completely explain the differences between the *in* vitro-effects observed.

It appears reasonable that this discrepancy may be due to other active constituents present in the extracts such as the hyperforins and the proanthocyanidins, which have also been shown to affect opioid receptors (cf. Zhu, M., Phillipson, D., Greengrass, P.M., Bowery, N.E. and Cai, Y., Phytochernistry 44 (1997), 441-447; Simmen, U., Berger, K., Burkard, W., Schaffher, W., Lundstrom, K., J. Recept. Signal Transduct. Res. 19(1-4) (1999), 59-74). The identification and quantification of these constituents seems however to be a very difficult task, since the chemical structures of most of these compounds are still unknown. In addition, proanthocyanidins may be separated only to a very limited degree by common HPLC analysis. Considering both the insufficient knowledge and the complexity of the antidepressant mechanism of action, standardization on a defined ingredient profile seems not very realistic. The standardization of total plant extracts on selected and specific biological activities by means of biological assays, in accordance with the invention and as described in Example 1, seems therefore to be a more convenient tool in order to qualify and rank *Hypericum perforatum* extracts and plant materials respectively according to their antidepressant effect. In accordance with the invention, the selected and identified elite plants can be developed to enhance further the specific biological activity and activities respectively of said elite plants.

The present invention provides for a method which allows the development and/or production of superior plants tailor-made for pharmaceutical and nutritional use. In particular, the present invention provides for a method which allows the identification, selection, breeding and/or cultivation of genetically superior plants, i.e. plants which produce and exhibit optimum contents of physiologically active metabolites and thus show an improved and enhanced biological activity in specified and specific biological assays. Moreover, the present invention provides for a method which allows to select genetically superior plants, i.e. elite plants having an enhanced specific biological activity, and to develop improved plant cultivation and processing technologies, i.e. technologies which lead to optimum contents of biologically active compounds in the plants by using biological assays, preferably biological assays *in vitro*.
A further advantage of the inventive methods is the early stage at which standardization, i.e. the provision of defined and standardized plants, takes place. Furthermore, the present invention allows to efficiently screen, e.g. a large number of genetically diverse accessions in order to select the optimal genotypes according to the desired pharmaceutical activity. Moreover, new individuals with an enhanced specific biological activity can be developed and created in accordance with the inventive methods.

## Claims

1. A method of enhancing a specific biological activity of a plant containing at least one physiologically active metabolite, comprising the steps of:
(a) collecting a plurality of samples of said plant;
(b) cultivating each of said samples under at least a first set of defined conditions to produce matured plants of each of said samples;
(c) processing each of said matured plants under at least a first set of defined conditions to obtain plant material of each of said matured plants;
(d) determining said specific biological activity of each of said plant material by means of at least one biological assay to obtain a ranking of said plant materials indicating plant materials with a higher specific biological activity;
(e) selecting said plant materials with a higher specific biological activity to identify elite plants; **characterized by** the steps of:
(f) multiplying at least one of said elite plants;
(h) cultivating each of said elite plants under at least a first set of defined conditions to produce matured plants of each of said elite plants;
(i) repeating said steps (c)-(f) with said matured plants of each of said elite plants;
(k) cultivating said elite plants under at least a first set of defined conditions to produce matured plants of said elite plants, and
(ii) repeating at least once a sequence of said steps (h) and (i) with variants produced from said elite plants.

2. The method of claim 1, wherein said step (ii) is repeated after said step (i) of claim 1 and prior to said step (k) of claim 1.

3. The method of clam 1, wherein said method further comprises the step of determining the genetic fingerprint of each of said elite plants, preferably before and after multiplication of said elite plants.

4. The method of claim 1, wherein said elite plants are cultivated by at least a first and a second set of defined conditions, preferably by a plurality of sets of defined conditions.

5. The method of claim 2, wherein said elite plants are cultivated by at least a first and a second set of defined conditions, preferably by a plurality of sets of defined conditions.

6. The method of claim 4, wherein said method further comprises the step of
(1) repeating at least once a sequence of said steps (c)-(f) and (k) of claim 1 with said matured plants of said elite plants of each previous sequence.

7. The method of claim 5, wherein said method further comprises the step of
(l) repeating at least once a sequence of said steps (c)-(f) and (k) of claim 1 with said matured plants of said elite plants of each previous sequence.

## Patentansprüche

1. Verfahren zur Verstärkung einer spezifischen Bioaktivität einer Pflanze, die mindestens einen physiologisch aktiven Metaboliten enthält, welches die Schritte:
(a) des Sammelns einer Mehrzahl von Proben der genannten Pflanze;
(b) der Kultivierung jeder Probe unter mindestens einem ersten Satz von definierten Bedingungen zur Erzeugung reifer Pflanzen aus jeder Probe;
(c) der Bearbeitung jeder reifen Pflanze unter mindestens einem ersten Satz von definierten Bedingung, um pflanzliches Material aus jeder reifen Pflanze zu erhalten;
(d) der Bestimmung der spezifischen Bioaktivität jedes pflanzlichen Materials mittels mindestens eines biologischen Tests, um eine Rangliste der Pflanzenmaterialien zu erhalten, die auf die Pflanzenmaterialien mit einer höheren spezifischen Bioaktivität hindeutet;
(e) der Auswahl des Pflanzenmaterials mit einer höheren spezifischen Bioaktivität zur Identifikation von Elitepflanzen umfasst,
**gekennzeichnet durch** die Schritte:
(f) der Vermehrung mindestens einer der Elitepflanzen;
(g) ---
(h) der Kultivierung jeder Elitepflanze unter einem ersten Satz von definierten Bedingungen zur Produktion von reifen Pflanzen aus jeder Elitepflanze;
(i) der Wiederholung der Schritte (c) - (f) mit reifen Pflanzen aus jeder Elitepflanze;
(k) der Kultivierung der Elitepflanzen unter mindestens einem ersten Satz von definierten Bedingungen zur Produktion von reifen Pflanzen aus den Elitepflanzen und
(ii) der mindestens einmaligen Wiederholung einer Sequenz der Schritte (h) und (i) mit aus den Elitepflanzen produzierten Varianten.

2. Verfahren nach Anspruch 1, wobei Schritt (ii) nach Schritt (i) von Anspruch 1 und vor Schritt (k) von Anspruch 1 wiederholt wird.

3. Verfahren nach Anspruch 1, wobei das Verfahren ferner den Schritt der Bestimmung des genetischen Fingerabdrucks jeder Elitepflanze, vorzugsweise vor und nach der Vermehrung der Elitepflanzen umfasst.

4. Verfahren nach Anspruch 1, wobei die Elitepflanzen nach mindestens einem ersten und einem zweiten Satz von definierten Bedingungen kultiviert werden, vorzugsweise nach einer Mehrzahl von Sätzen von definierten Bedingungen.

5. Verfahren nach Anspruch 2, wobei die Elitepflanzen nach mindestens einem ersten und einem zweiten Satz von definierten Bedingungen kultiviert werden, vorzugsweise nach einer Mehrzahl von Sätzen von definierten Bedingungen.

6. Verfahren nach Anspruch 4, wobei das Verfahren ferner den Schritt
(1) der Wiederholung von mindestens einer Sequenz der Schritte (c) - (f) und (k) von Anspruch 1 mit den reifen Pflanzen aus den Elitepflanzen jeder vorangegangenen Sequenz umfasst.

7. Verfahren nach Anspruch 5, wobei das Verfahren ferner den Schritt
(1) der Wiederholung mindestens einer Sequenz der Schritte (c) - (f) und (k) von Anspruch 1 mit den reifen Pflanzen aus den Elitepflanzen jeder vorangegangenen Sequenz umfasst.

## Revendications

1. Procédé d'amélioration d'une activité biologique spécifique d'une plante contenant au moins un métabolite physiologiquement actif, comprenant les étapes consistant:
(a) à recueillir plusieurs échantillons de ladite plante;
(b) à cultiver chacun desdits échantillons sous au moins un premier ensemble de conditions définies pour produire des plantes mûries de chacun desdits échantillons;
(c) à traiter chacune desdites plantes mûries sous au moins un premier ensemble de conditions définies pour obtenir une matière de plante de chacune desdites plantes mûries;
(d) à déterminer ladite activité biologique spécifique de chacune desdites matières de plante au moyen d'au moins un essai biologique pour obtenir une évaluation desdites matières de plante indiquant les matières de plante ayant une activité biologique spécifique plus élevée;
(e) à choisir lesdites matières de plante ayant une activité biologique spécifique plus élevée pour identifier des plantes élites;
**caractérisé par** les étapes consistant:
(f) à multiplier au moins l'une desdites plantes élites;
(h) à cultiver chacune desdites plantes élites sous au moins un premier ensemble de conditions définies pour produire des plantes mûries de chacune desdites plantes élites;
(i) à répéter lesdites étapes (c) à (f) avec lesdites plantes mûries de chacune desdites plantes élites;
(k) à cultiver lesdites plantes élites sous au moins un premier ensemble de conditions définies pour produire des plantes mûries desdites plantes élites; et
(ii) à répéter au moins une fois une séquence desdites étapes (h) et (i) avec des variantes produites à partir desdites plantes élites.

2. Procédé selon la revendication 1, ladite étape (ii) est répétée après ladite étape (i) de la revendication 1 et avant ladite étape (k) de la revendication 1.

3. Procédé selon la revendication 1, ledit procédé comprenant en outre l'étape consistant à déterminer l'empreinte génétique de chacune desdites plantes élites, de préférence avant et après la multiplication desdites plantes élites.

4. Procédé selon la revendication 1, dans lequel lesdites plantes élites sont cultivées d'après au moins un premier et un second ensemble de conditions définies, de préférence d'après plusieurs ensembles de conditions définies.

5. Procédé selon la revendication 2, dans lequel lesdites plantes élites sont cultivées d'après au moins un premier et un second ensemble de conditions définies, de préférence d'après plusieurs ensembles de conditions définies.

6. Procédé selon la revendication 4, ledit procédé comprenant en outre l'étape consistant
(1) à répéter au moins une fois une séquence desdites étapes (c) à (f) et (k) de la revendication 1 avec lesdites plantes mûries desdites plantes élites de chaque séquence précédente.

7. Procédé selon la revendication 5, ledit procédé comprenant en outre l'étape consistant
(l) à répéter au moins une fois une séquence desdites étapes (c) à (f) et (k) de la revendication 1 avec lesdites plantes mûries desdites plantes élites de chaque séquence précédente.
